# EUROPEAN PATENT APPLICATION

(11) **EP 2 745 873 A1**
(43) Date of publication of application: **25.06.2014**
(21) Application number: 13179447.1
(22) Date of filing: 06.08.2013
(51) Int. Cl.: A61N 1/05, A61N 1/08

(54) **An electronic module for a system for neural applications**

(30) Priority: 21.12.2012 US 201261740986 P; 21.12.2012 EP 12199296
(71) Applicant: Sapiens Steering Brain Stimulation B.V., 5656 AE Eindhoven (NL)
(72) Inventor: Tol, Jeroen Jacob Arnold, 5645 JG Eindhoven (NL); Young, Edward Willem Albert, 6211 LN Maastricht (NL); Bakker, Egbertus Johannes Maria, 4261 BX Wijk en Aalburg (NL)
(74) Representative: Prinz & Partner

(57) **Abstract**

The present invention relates to an electronic module (500) for a system for neural applications (100), especially a neurostimulation and/or neurorecording system (100), further especially a deep brain stimulation (DBS) system (100), wherein the electronic module (500) comprises at least one integrated passive device (560). Furthermore, the present invention relates to a lead (300) for neural stimulation, a controller (110), an active lead can element (111) and a system for neural applications (100), especially a system (100) for neurostimulation and/or neurorecording applications, for instance a deep brain stimulation system (100).

## Description

The present invention relates to an electronic module for a system for neural applications, a lead for neural stimulation, a controller, an active lead can element and a system for neural applications, especially a system for neurostimulation and/or neurorecording applications, for instance a deep brain stimulation system.

Implantable neurostimulation devices have been used for the past ten years to treat acute or chronic neurological conditions. Deep brain stimulation (DBS), the mild electrical stimulation of sub-cortical structures, belongs to this category of implantable devices, and has been shown to be therapeutically effective for Parkinson's disease, Dystonia, and Tremor. New applications of DBS in the domain of psychiatric disorders (obsessive compulsive disorder, depression) are being researched and show promising results. In existing systems, the probes are connected to an implantable current pulse generator.

Currently, systems are under development with more, smaller electrodes in a technology based on thin film manufacturing. These novel systems consist of a lead made from a thin film based on thin film technology, as e.g. described in WO 2010/055453 A1. The thin film leads are fixed on a core material to form a lead. These probes will have multiple electrode areas and will enhance the precision to address the appropriate target in the brain and relax the specification of positioning. Meanwhile, undesired side effects due to undesired stimulation of neighbouring areas can be minimized.

Leads that are based on thin film manufacturing are e.g. described by US 7,941,202 and have been used in research products in animal studies.

In existing systems, the DBS lead has e.g. four 1.5 mm-wide cylindrical electrodes at the distal end spaced by 0.5 mm or 1.5 mm. The diameter of the lead is 1.27 mm and the metal used for the electrodes and the interconnect wires is an alloy of platinum and iridium. The coiled interconnect wires are insulated individually by fluoropolymer coating and protected in an 80 micron urethane tubing. With such electrode design, the current distribution emanates uniformly around the circumference of the electrode, which leads to stimulation of all areas surrounding the electrode.

The lack of fine spatial control over field distributions implies that stimulation easily spreads into adjacent structures inducing adverse side-effects in about 30% of the patients. To overcome this problem, systems with high density electrode arrays are being developed, hence providing the ability to steer the stimulation field to the appropriate target.

The clinical benefit of DBS is largely dependent on the spatial distribution of the stimulation field in relation to brain anatomy. To maximize therapeutic benefits while avoiding unwanted side-effects, precise control over the stimulation field is essential.

During stimulation with existing DBS leads there is an option to use monopolar, bipolar, or even tripolar stimulation. Neurostimulator devices with steering brain stimulation capabilities can have a large number of electrode contacts (n > 10) that can be connected to electrical circuits such as current sources and/or (system) ground. Stimulation may be considered monopolar when the distance between the anode and cathode is several times larger than the distance of the cathode to the stimulation target. During monopolar stimulation in homogeneous tissue the electric field is distributed roughly spherical similar to the field from a point source. When the anode is located close to the cathode the distribution of the field becomes more directed in the anode-cathode direction. As a result the field gets stronger and neurons are more likely to be activated in this area due to a higher field gradient.

The mechanisms of DBS are unknown. However, it is hypothesized that polarization (de- and/or hyperpolarization) of neural tissue is likely to play a prominent role for both suppression of clinical symptoms, as well as induction of stimulation-induced side-effects. In order to activate a neuron it has to be depolarized. Neurons are depolarized more easily close to the cathode than by the anode (about 3-7 times more depending on type of neuron, etc.).

Therefore, compared to monopolar stimulation the effect of bipolar stimulation is less spread of the electric field, a stronger electric field between the anode and cathode, and more activated neurons close to the cathode. Bipolar stimulation is therefore used to focus the field to certain areas in cases when beneficial stimulation is not obtained during monopolar stimulation.

DBS leads are typically implanted via a stereotactic neurosurgical procedure. The planning of a stereotactic procedure involves the identification of the DBS target (e.g. the subthalamic nucleus) on the basis of the MR or CT images of the patient's head/brain and defining a point within the target nucleus. Eventually the stereotactic planning station (e.g. a computer system) provides the stereotactic coordinates of the target point. The stereotactic coordinates can be referenced externally and thus be used in the operating room to precisely navigate the DBS lead to the selected point in the brain.

To reduce the clinical impact of the implanted parts on a patient, a miniaturization of the components of the DBS system like the implantable pulse generator and e.g. the active lead can element comprising electronic parts of the DBS system is not only desirable but also mandatory. The active lead can element has strict size requirements to reduce its clinical impact, for example skin erosion, when the active lead can element is mounted on a patient's skull. The implantable pulse generator cannot be made too large either, although desired from a battery longevity point of view, because of patient comfort and displacement risk if the implantable pulse generator size and weight is made too large.

It is therefore an object of the present invention to improve an electronic module for a system for neural applications, a lead for neural stimulation, a controller, an active lead can element and a system for neural applications, in particular in that a reduction of the passive electronic components volume and area claim of at least a part of the electronics of a system for neural applications can be provided.

The above object is solved according to the present invention by an electronic module for a system for neural applications, especially a neurostimulation and/or neurorecording system, further especially a deep brain stimulation (DBS) system, wherein the electronic module comprises at least one integrated passive device.

By this, the advantage is achieved that a reduction of the passive electronic components volume and area claim of at least a part of the electronics of a system for neural applications can be provided.

In particular, a significant reduction of the dominant volume claim of passive electrical components and networks of an implant can be achieved with the application of integrated passive devices, also referred to as integrated passive components. This integration brings the passives volume claim in line with the achievable mechanical miniaturization leading to maximum shrinkage of the volume and area of a system for neural applications.

Filters and DC blocking capacitors are the discrete components that claim most volume and area in the active lead can because of the large number of connections of the active lead can electronics with the outside world, and therefore, with a high number of feed-through pins. A reduction of the passives volume and area claim is essential to meet the strict size requirements of the electronics of a system for neural applications, which can be achieved by integration of the passives into a single module.

This passives integration can be achieved with various integrated passive device technologies. Capacitor modules with an array of multilayer ceramic capacitors are available on the market. Integration of different types of passives (resistors, capacitors and inductors) with one or more interconnect layers using thin or thick film technology to realize the integration of multiple passives on a common ceramic substrate is quite common. Recently, integrated passive devices with several integrated capacitors and interconnections on a silicon chip have also become available.

Integrated passive devices or integrated passive components are attracting an increasing interest due to constant needs e.g. of handheld wireless devices to further decrease in size and cost and increase in functionality. Many functional blocks such as impedance matching circuits, harmonic filters, couplers and baluns and power combiner/divider can be realized by integrated passive devices technology. Integrated passive devices can be designed as flip chip mountable or wire bondable components and the substrates for integrated passive devices usually are thin film substrates like silicon, alumina or glass.

Integrated passive devices are generally fabricated using standard screen print technology on ceramic or standard wafer fab technologies on silicon such as thin film and photolithography processing. Alternatively, modules can be manufactured from green foil e.g. with Low Temperature Co-fired Ceramic technology. DC blocking capacitors are often applied in implantable medical devices such as a system for neural applications in order to prevent e.g. DC leakage currents. Those and other passive components can be integrated on an integrated passive device, for example, to save precious implant volume and/or increase component reliability, which becomes attractive if many passives are needed. Recently, the DC blocking capacitors can also be realised in an integrated passive device where the capacitors are integrated on a silicon chip, for example, 3D capacitors in a common substrate.

For this type of integrated passive device, if a DC bias voltage is applied to prevent forward biasing of the capacitor-substrate diodes during normal operation, a failure of the integrated passive device can give rise to tissue leakage current. For example, if the DC blocking capacitors are directly connected to e.g. a stimulation and/or recording electrode lead, an integrated passive device failure might short the DC biased integrated passive device chip substrate to one or more lead electrodes directly. This failure might lead to unwanted and potentially hazardous tissue DC leakage current. Advantageously, due to the use or provision of a leakage current detection and/or prevention means the integrated passive device can be biased safely.

The electronic system may be an electronic system for a system for neural applications or, more specifically for brain applications, preferably for a neurostimulation and/or neurorecording system. Such a neurostimulation and/or neurorecording system may be e.g. a DBS system.

The lead may e.g. comprise at least one thin film, whereby the thin film comprises a proximal end and a distal end, the lead further comprising a plurality of electrodes on the distal end of the thin film.

The thin film may include at least one electrically conductive layer, preferably made of a biocompatible material. The thin film may be assembled to the carrier and further processed to constitute the lead element. The thin film for a lead is preferably formed by a thin film product having a distal end, a cable with metal tracks and a proximal end. The distal end of the thin film may be forming a part of the distal end of the lead or merely the distal end of the lead.

The distal end of the lead may be the end of the lead, which is in the implanted state of the lead the remote end of the lead with regard to the body surface area. In particular, in case of a lead for brain application, the distal end of the lead is the lower end of the lead, which is remote to the burr-hole of the skull, through which the lead is implanted.

The active lead can element may comprise electronic means to address the plurality of electrodes and at least one active lead can connecting means. Further, the active lead can element may be hermetically or merely hermetically sealed and may comprise electronic means to address the plurality of electrodes on the distal end of the thin film, which is arranged at the distal end and next to the distal tip of the lead. The plurality of electrodes may comprise 5-10 or more electrodes, e.g. 16 or 32 electrodes, or in preferred embodiments e.g. 40 electrodes or more. The electrodes may be arranged such that the electrodes are merely evenly distributed arranged all over the distal end of the lead.

The active lead can element may be e.g. basically a multi-pin connector with a low-count feed-through (LCFX) connector with e.g. five pins for the interface cable e.g. between active lead can element and implantable pulse generator and another high-count feed-through (HCFX) connector with e.g. 40 pins for the lead. Any other suitable number of pins for the LCFX and HCFX may be also used if needed.

For instance, it is mechanically possible to design these two feed-through connectors with a high pin density to reduce the active lead can element area significantly. However, this area advantage can only materialize if the electrical components of the active lead can element can be shrunk in similar proportions as the feed-through connectors, which is now advantageously possible according to the invention. Moreover, e.g. a very thin active lead can element, most desirable to reduce its impact on skin erosion, not only requires a high pin density but also a reduction in the height of both feed-through pins and interior electrical components. Thus both the active lead can element electronics volume and area must be miniaturized to realize a small active lead can element. The techniques to shrink the active lead can element can also advantageously be applied to the implantable pulse generator, or any other implant module, for example, to trade for an increase in battery volume to extend its capacity and/or for increasing the functionality of the implantable pulse generator.

Furthermore, it is possible that the electronic module comprises at least one filtering element, wherein the filtering element is exemplarily a feed-through filter, and/or at least one blocking element, wherein the blocking element is exemplarily a DC blocking element and/or at least one application specific integrated circuit (ASIC).

Especially, it is possible that the integrated passive device comprises the at least one filtering element, wherein the filtering element is exemplarily a feed-through filter, and/or the at least one blocking element, wherein the blocking element is exemplarily a DC blocking element.

It is for instance possible that the filtering element is realized in a first integrated passive device and that the blocking element is realized in a second integrated passive device.

The filtering element and/or the blocking element and/or the ASIC may be stacked one upon the other. The stack may be a thin stack and a low volume. The filtering can be provided by any means which is/are capable to provide a filtering. In particular, a filtering can be provided by any passive means or passive network.

The filtering element may be configured such that interferences, in particular unwanted interferences e.g. caused by mobile phones or the like, can be removed before they may enter e.g. a part of the housing of the system for neural applications. Thereby, the advantage is achieved that a protection against electromagnetic interference (EMI) is provided, for example, against mobile phone induced fields while the patient is using its mobile phone. The filtering element may be or may comprise e.g. an RF feed-through filter. The filter may comprise e.g. a capacitor, a coil, an inductor, a resistor or any other suitable passive component.

The blocking element may be configured such that in the event of a leakage current such leakage current, in particular DC leakage current flow is prevented. Regulations demand that (almost) no DC current flows through the patient carrying an implant such as a deep brain stimulator, even when a (single) failure occurs of, for example, the implant's electronics. This DC leakage design problem is solved by the application of DC blocking capacitors. Again, with a high number of feed-through pins, it becomes mandatory to integrate those blocking capacitors to achieve a minimum volume and area claim as opposed to the application of discrete components.

The ASIC may comprise a part or e.g. all active electronics with some external passives, for example, power supply decoupling capacitors. A substrate with integrated passives (resistors, capacitors and inductors) may be used as substrate for off-chip (rerouting to) an ASIC and together a so called hybrid or hybrid integrated circuit may be realized. By the use of one or more ASICs the active electronic components of at least a part of the system for neural application may be miniaturized.

Mechanical miniaturization of an implant module really pays off if all of its interior electrical components can be shrunk to the same extent. Active electronics can be miniaturized by integration on one or more ASIC dies but the largest part of an implant's volume is often claimed by the external (passive) components and networks that cannot be integrated into the ASIC because
1. it is technologically not feasible, for example, the integration of inductors or capacitors with large values cannot be integrated on-chip;
2. it is too costly, because the desired components, for example, hundreds of nano Farad DC blocking capacitors, consume too much chip area;
3. it is functionally undesired or ineffective to put the passives on the ASIC die, for example, feed-through filters are only effective when put close to and/or integrated with the feed-through pins, while DC blocking capacitors should electrically be isolated from the active electronics by integration on a separate die and/or substrate.

The volume and area claim of the passive components is especially high if feed-through connectors have a high pin density and feed-through filter networks are required. However, by the combination of integrated passive devices and one or more ASICs the advantage can be achieved that not only the active electronics can be shrunk due to the use of ASICs but also the passive electronics can be shrunk due to the use of integrated passive devices.

Moreover, it is possible that the integrated passive device comprises and/or is connected and/or connectable to several sections of interconnects e.g. one or more low-count feed-through pins and/or one or more high-count feed-through pins and/or an ASIC and/or another integrated passive device and/or at least one bias terminal, especially wherein the in- and/or output of the integrated passive device is connected to the filtered out- and/or input via at least one capacitor and/or at least one resistor and/or at least one inductor, and/or that the integrated passive device comprises a substrate and/or at least one diode and at least one passive electronic component which is arranged on and/or in the substrate.

The filtering of the filtered out- and/or input may be provided by the at least one filtering element.

Additionally, it is possible that the electronic module comprises at least one first connector element, e.g. the LCFX connector, and at least one second connector element, e.g. the HCFX connector, the first connector element being configured such that the electronic module is directly and/or indirectly connectable or connected to a controller, e.g. the integrated passive device, which is at least configured to supply and/or provide and/or measure at least one voltage and/or at least one current and/or at least one voltage waveform and/or at least one current waveform especially via one or more stimulation and/or recording and/or clock and/or power and/or communications outputs and/or inputs, the second connector element being configured such that the electronic module is directly and/or indirectly connectable or connected to a lead for neural stimulation and/or recording and/or the electronic module is directly and/or indirectly connectable or connected to an active lead can element and/or directly and/or indirectly connectable or connected to a housing.

The electronic module and/or its components advantageously make sure that, to the largest extent possible, any electromagnetic interference (EMI) is kept outside the area of the active lead can that is vulnerable to EMI, while EMI generated inside the active lead can is also prevented to radiate to the outside. A conductive (e.g. titanium) housing is used to achieve this electromagnetic shielding with the electromagnetic opening, formed by the (non-conductive) ceramic with the embedded LCFX and HCFX pins, being electromagnetically closed by the feed-through filtering element of the electronic module. This filtering element, exemplarily an array of feed-through capacitors, redirects the EMI currents to the (conductive) housing.

Furthermore, it is possible that the integrated passive device comprises one or more passive electronic components, especially at least one capacitor and/or at least one inductor and/or one diode and/or at least one substrate terminal, further especially only capacitors and/or inductors and/or diodes and/or at least one substrate terminal, especially only one or more passive electronic components, especially at least one capacitor and/or at least one inductor and/or at least one diode and/or at least one substrate terminal, further especially only capacitors and/or inductors and/or diodes and/or at least one substrate terminal.

Moreover, the electronic system comprises exemplarily at least one biasing means.

If the integrated passive device has its components embedded in and/or integrated on a common (silicon) chip substrate, the substrate may need to be biased with a voltage that is lower (for a p-type substrate but higher for an n-type substrate) than the lowest (highest for n-type substrate) voltage ever appearing on the in- and/or output terminals of the integrated passive device so that all component-substrate junctions remain reversed biased during normal operation. Otherwise unwanted substrate currents are injected into the integrated passive device, and therefore, into the integrated passive device components. Thus this type of integrated passive device, whose components share a common conductive substrate, should be provided with a DC voltage to bias its substrate correctly. Especially (substrate) diodes of the integrated passive device may be biased, respectively reverse biased.

Moreover, it is possible that the integrated passive device comprises one or more feed-through capacitors which are mounted on and/or integrated into a substrate, in particular a ceramic substrate and/or one or more integrated DC blocking capacitors which are mounted on and/or integrated into a substrate, in particular a silicon substrate. Alternatively, the integrated passive component is manufactured from
- a flexible organic capacitor in a flex foil and/or PCB, and/or
- a (screen) printed capacitor on a ceramic substrate, and/or
- a capacitor built using thick film on ceramic technology, and/or
- a capacitor that is built on a ceramic substrate using physical vapor deposition (PVD), and/or
- a stack of ceramic substrates with a (screen) printed thick film capacitor, in particular a capacitor built using low temperature co-fired ceramic (LTCC) technology, and/or
- a 3D-in-silicon capacitor.

The integrated passive component may also be manufactured from a metal-insulator-metal (MIM) capacitor on silicon technology.

The one or more feed-through capacitors may be a part of the feed-through filter. The feed-through capacitors may be (screen) printed e.g. on the top side of the substrate of the integrated passive device. The capacitors may be made of a sandwich of two (or more) conductive layers with a (relatively) high-k dielectric in between. The one or more integrated DC blocking capacitors may be a part of the at least one blocking element.

Also, it is possible that the substrate comprises a contact surface portion, especially a ground plane on the substrate top, which is directly and/or indirectly electrically and/or capacitively connected to a (conductive) housing of the electronic module, especially via a ring of conductive adhesive, preferably conductive adhesive epoxy, so that the feed-through capacitors and the housing together form a (high-frequency) closed, miniaturized Faraday cage only penetrated by feed-through pins that are capacitively and/or electrically coupled to the Faraday cage.

The housing of the electronic module may be a conductive housing, for example, a titanium housing. A metal housing combines mechanical protection of the module with electromagnetic shielding. Alternatively, a metalized polymer can be used for mechanical protection and electromagnetic shielding.

By this, the advantage is achieved that, to the largest extent possible, any electromagnetic interference (EMI) is kept outside the area of the active lead can that is vulnerable to EMI, while EMI generated inside the active lead can is also prevented to radiate to the outside. Each feed-through pin may connect to the top contact (e.g. a gold top contact) of a single capacitor in the array of the feed-through capacitors (e.g. a thick film capacitor array) via stud bumps. Through-hole signal vias may connect the feed-through pins to one or more interconnect layers on the bottom side of the substrate. Each metal layer can have a maximum area fill factor so that a maximum overlap between all metal layers is achieved, increasing the capacitance per printed dielectric layer, and forming a (high-frequency) closed Faraday cage with the (conductive) housing of the module.

Thus a minimum spacing between the individual top contacts and a minimum spacing between the through-hole vias and the ground plane increase the filter's efficacy, because it increases the capacitance per pin and more importantly, it minimizes the openings in the metal layers. If desired, an additional ground layer can be applied to electrically close the filter's 3D structure further. Further, the mutual ground plane of all array capacitors may extend beyond the capacitor arrays itself and may be connected with a ring of ground vias to the other side of the substrate to provide ground to the interior of the ALC. Alternatively or additionally, the top and bottom substrate ground planes can be connected via metallization wrapped around the substrate's side edges. So, the ground plane on the substrate top is electrically connected to a (conductive) housing of the electronic module via a ring of conductive adhesive, especially conductive adhesive epoxy, so that the feed-through capacitors and the housing together form a (high-frequency) closed, miniaturized Faraday cage only penetrated by the feed-through pins from which EMI currents are diverted through feed-through capacitors to the (conductive) housing before they can enter the housing. The other way around, this embedding of the feed-through filter in the (conductive) housing guarantees, to the largest extent possible, that any EMI generated inside the active lead can is also prevented to radiate to the outside.

Furthermore, it is possible that the substrate comprises holes, wherein through-hole signal vias connect the feed-through pins to one or more interconnect layers on the bottom side of the substrate.

Additionally, it is possible that the electronic module comprises at least partially a multi-layer structure, wherein especially the filtering element forms a first layer and/or the blocking element forms a second layer and/or the ASIC forms a third layer.

By this multi-layer structure the advantage is achieved that all or almost all electrical and electronic components fit in a very small volume and area.

Moreover, it is possible that the module comprises a dedicated routing substrate and/or an already available substrate for routing for passive components which are mounted outside an integrated passive device, in particular for at least one discrete component, e.g. a surface mounted device (SMD).

The routing substrate may be the substrate which carries the DC blocking elements, in particular the DC blocking capacitors.

More efficient filtering, for example better EMI suppression, can be realized with a combination of passive components, capacitors, resistors and inductors. Further, it is possible that the integrated passive device, for example realizing the feed-through filtering element, comprises at least one resistor, especially a series resistor, and/or an inductor, especially a series inductor, and/or a capacitor, especially a parallel capacitor, wherein the resistor and/or the inductor and/or the capacitor is/are configured such that a (parasitic) filter resonance may be dampened and/or the filter's suppression, especially the suppression of electromagnetic interference, may be improved and/or increased.

For example, a series resistor and/or an inductor and/or a capacitor can be added to dampen any (parasitic) filter resonances and/or to increase the (series) impedance of the filtering element towards the electronics it is connected to. By this, the (high-frequency) shorting efficacy of the filter to the housing may be improved.

Also, the present invention relates to a lead with the features of claim 12. Accordingly, a lead is provided, the lead being a lead for neural stimulation comprising at least one electronic module for a system for neural applications according to any of claims 1 to 11.

Furthermore, the present invention relates to a controller with the features of claim 13. Accordingly, a controller is provided, especially an implantable pulse generator comprising at least one electronic module for a system for neural applications according to any of claims 1 to 11.

Additionally, the present invention relates to an active lead can element with the features of claim 14. Accordingly, an active lead can element comprises at least one electronic module for a system for neural applications according to any of claims 1 to 11.

Moreover, the present invention relates to a system for neural applications with the features of claim 15. Accordingly, a system for neural applications is provided, especially a system for neurostimulation and/or neurorecording applications, especially a deep brain stimulation system. The system for neural applications comprises at least one electronic module for a system for neural applications according to any of claims 1 to 11 and/or comprises at least one lead according to claims 12 and/or comprises at least one controller according to claim 13 and/or comprises at least one active lead can element according to claim 14.

Further details and advantages of the present invention shall be described hereinafter with respect to the drawings:
- Fig. 1:: a schematical drawing of a neurostimulation system for deep brain stimulation (DBS);
- Fig. 2:: a further schematical drawing of a probe of a neurostimulation system for deep brain stimulation (DBS) and its components;
- Fig. 3:: a schematical drawing of a probe system according to the present invention;
- Fig. 4:: a schematical drawing of a probe system according to the present invention with an electronic module;
- Fig. 5:: a schematical top X-ray view of the electronic module showing the top feedthrough and the underlying feedthrough capacitor of the integrated passive device;
- Fig. 6:: a schematical cross-sectional view of the electronic module according to a first embodiment;
- Fig. 7:: a schematical cross-sectional view of the electronic module according to a second embodiment;
- Fig. 8:: a schematical cross-sectional view of the electronic module according to a third embodiment;
- Fig. 9:: a schematical cross-sectional view of the electronic module according to a fourth embodiment;
- Fig. 10:: a schematical cross-sectional view of a feed-through capacitor in thick film technology;
- Fig. 11:: a schematical view of the electronic module within an active lead can;
- Fig. 12:: a further schematical view of the electronic module within an active lead can as shown in Fig. 11; and

- Fig. 13:: a schematic drawing of a cross-section of an integrated passive device with multiple (floating) junction isolated (trench) DC blocking capacitors in a common substrate (left) with equivalent electrical circuit (right).

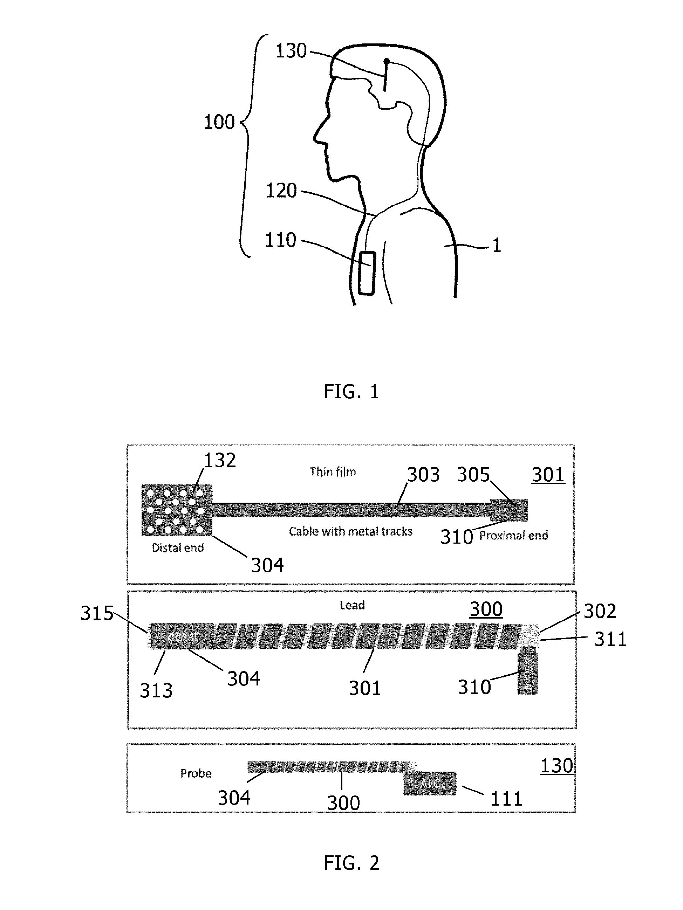

A possible embodiment of a neurostimulation system 100 for deep brain stimulation (DBS) is shown in Figure 1. The neurostimulation system 100 comprises at least a controller 110 that may be surgically implanted in the chest region of a patient 1, typically below the clavicle or in the abdominal region of a patient 1. The controller 110 can be adapted to supply the necessary voltage pulses. The typical DBS system 100 may further include an extension wire 120 connected to the controller 110 and running subcutaneously to the skull, preferably along the neck, where it terminates in a connector. A DBS lead arrangement 130 may be implanted in the brain tissue, e.g. through a burr-hole in the skull.

Figure 2 further illustrates a typical architecture for a Deep Brain Stimulation probe 130 that comprises a DBS lead 300 and an active lead can element 111 comprising electronic means to address electrodes 132 on the distal end 304 of the thin film 301, which is arranged at the distal end 313 and next to the distal tip 315 of the DBS lead 300. The lead 300 comprises a carrier 302 for a thin film 301, said carrier 302 providing the mechanical configuration of the DBS lead 300 and the thin film 301. The thin film 301 may include at least one electrically conductive layer, preferably made of a biocompatible material. The thin film 301 is assembled to the carrier 302 and further processed to constitute the lead element 300. The thin film 301 for a lead is preferably formed by a thin film product having a distal end 304, a cable 303 with metal tracks and a proximal end 310. The proximal end 310 of the thin film 301 arranged at the proximal end 311 of the lead 300 is electrically connected to the active lead can element 111. The active lead can element 111 comprises the switch matrix of the DBS steering electronics. The distal end 304 comprises the electrodes 132 for the brain stimulation. The proximal end 310 comprises the interconnect contacts 305 for each metal line in the cable 303. The cable 303 comprises metal lines (not shown) to connect each distal electrodes 132 to a designated proximal contact 305.

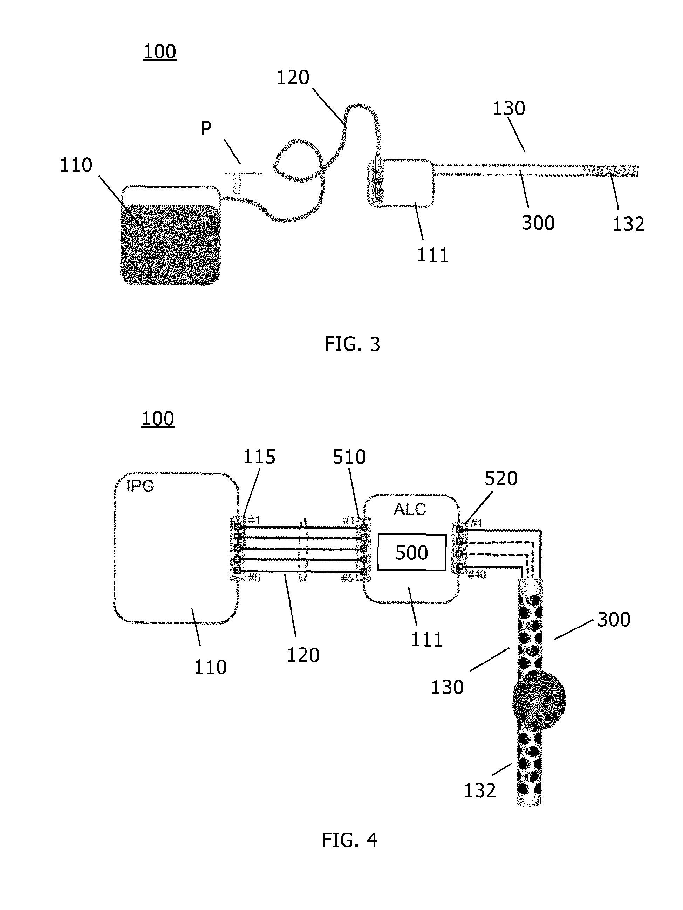

Figure 3 shows schematically and in greater detail an embodiment of a system 100 for brain applications, here for neurostimulation and/or neurorecording as a deep brain stimulation system 100 as shown in Figures 1 and 2. The probe system 100 comprises at least one probe 130 for brain applications with stimulation and/or recording electrodes 132, wherein e.g. 40 electrodes 132 can be provided on outer body surface at the distal end of the probe 130. By means of the extension wire 120 pulses P supplied by controller 110 can be transmitted to the active lead can 111. The controller 110 can be an implantable pulse generator 110.

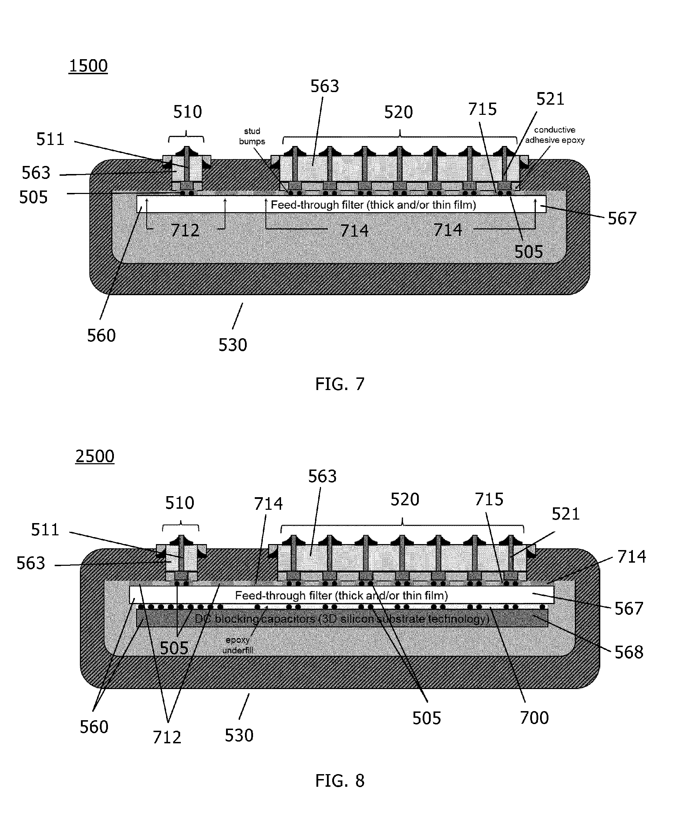

Figure 4 shows a schematical drawing of a system 100 for brain applications according to the present invention with an electronic module 500. The electronic module 500 (which can be also an electronic module 1500, 2500, 3500 as shown in Figures 7 to 9) is in this embodiment integrated into the active lead can 111.

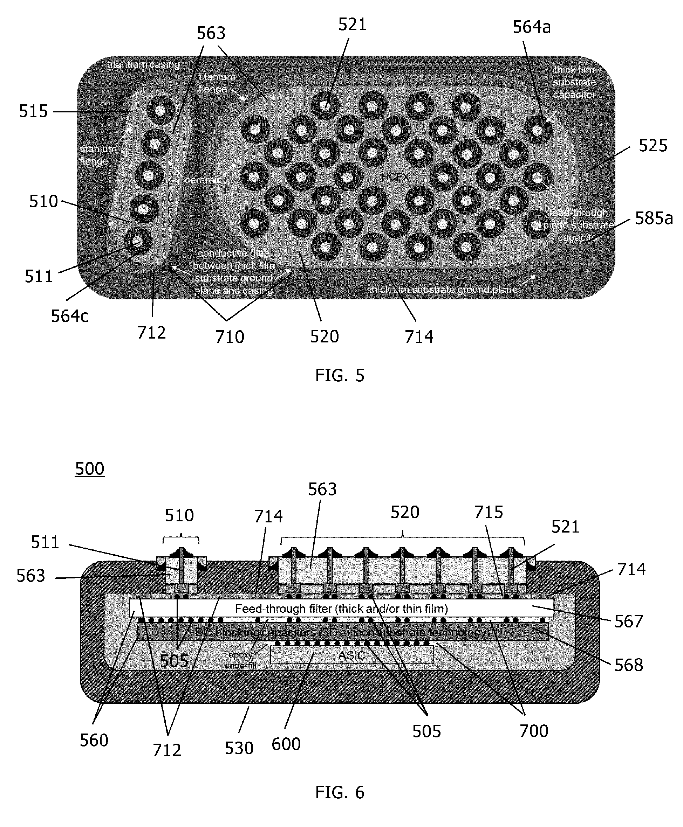

As can be seen in the embodiments shown in Figures 6 to 9 of the possible embodiments of the electronic module 500, 1500, 2500, 3500, the electronic module 500, 1500, 2500, 3500 comprises at least one integrated passive device 560.

The system 100 having a DBS probe 130 as defined above comprises an IPG 110 and an active lead can 111 with an array of electronic switches that connects electrodes 132 arranged at the distal end of the lead 300 with the pulse generator lines of the implantable pulse generator 110. In addition, it includes neural recording facilities. IPG 110 and active lead can 111 are connected through an interface cable 120 with, here for example, five lines. Accordingly, the implantable pulse generator 110 has a 5-pin LCFX connector 115 which is connected via the interface cable 120 with the 5-pin LCFX connector 510 of the active lead can 111.

The active lead can 111 comprises a multi-pin connector with a 5-pin LCFX connector 510 for the interface cable 120 and a 40-pin HCFX connector 520 for the lead 300. It is mechanically possible to design these two feed-through connectors 510, 520 with a high pin density to reduce the area of the active lead can 111 significantly. However, this area advantage can only materialize if the electrical components of the active lead can 111 can be shrunk in similar proportions as the feed-through connectors 510, 520. Moreover, a very thin active lead can 111, most desirable to reduce its impact on skin erosion, not only requires a high pin density but also a reduction in the height of both feed-through pins 511, 521 and interior electrical components. Thus both the electronics volume and area of the active lead can 111 are miniaturized to realize a small active lead can 111.

Note that techniques to shrink the active lead can 111 can also advantageously be applied to the implantable pulse generator 110, or any other implant module, for example, to trade for an increase in battery life time and/or increased functionality.

Figure 5 shows an X-ray picture of the electronic module 500. In particular, Figure 5 shows the top feedt hrough and the underlying feed through capacitor of the integrated passive device. The embodiments of the electronic module 1500, 2500, 3500 shown in Figures 7 to 9 may be embodied similarly.

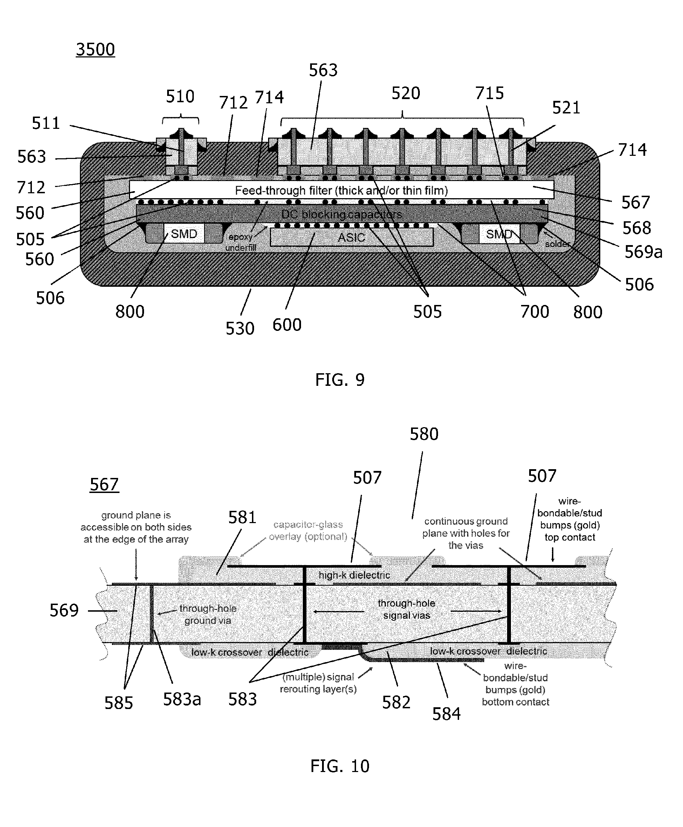

In Figure 5, both the LCFX connector 510 and the HCFX connector 520 on the outside as well as the top of the substrate 568 (see e.g. Figure 10) of the capacitor array on the inside, underneath the feedthrough, are shown. The ground plane 585 (see Figure 10) of the capacitor array is electrically connected via the top part 585a of the ground plane to the housing 530 of the active lead can element 111 via a ring of conductive epoxy glue 710.

In particular, the LCFX connector 510 is directly and/or indirectly electrically and/or capacitively connected to the housing 530 of the active lead can 111 via a ring of conductive epoxy glue 712 and the HCFX connector 520 is electrically connected to the housing 530 of the active lead can 111 via a ring of conductive epoxy glue 714. It is also possible to apply a single ring of conductive epoxy glue surrounding both LCFX and HCFX connectors to electrically connect the housing 530 to the top ground plane 585a. If required, the feed through pins can be grouped into other combinations, in multiple sections or combined into just one single feed through.

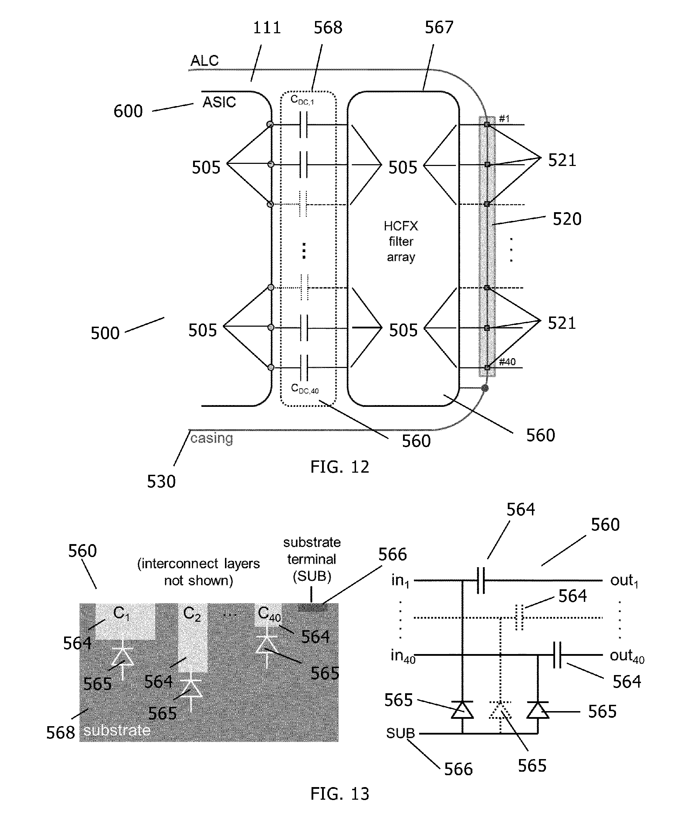

The electronic module 500 (likewise the electronic modules 1500, 2500, 3500 of Figures 7 to 9) comprises HCFX connection pins 521 and LCFX connection pins 511. The HCFX connection pins 521 are connected to the DC blocking capacitors 564 of integrated passive device 568 (see e.g. Figure 12 and 13) and also to the feed-through filter capacitors 564a of integrated passive device 560 (see e.g. Figure 6 to 12). Although not explicitly shown, the LCFX connection pins 511 are also connected to integrated passive device 568 and integrated passive device 560 to realize the same DC blocking and EMI filtering for the LCFX.

Each feed-through pin 511, 521 contacts to a capacitor top contact 507 (see Figure 10), which can be a gold top contact, on the substrate top of thick film integrated passive device 567. The HCFX pins 521 are contacted to capacitors 564a and the LCFX pins 511 are contacted to capacitors 564c. The top contacts 507 are simultaneously the top plates of capacitors 564a and capacitors 564c (see Figure 10). Thus a thick film substrate with screen printed capacitors 564a and capacitors 564c forms a (single) feed-through filter substrate 567 (see e.g. Figures 6 to 12) that is directly put on top of and connected with the feed-through pins 511, 521.

The LCFX connector 510 has a titanium flange 515 forming a border of the LCFX connector 510 and the HCFX connector 520 has a titanium flange 525 forming a border of the HCFX connector 520. The titanium flanges are integrated in the titanium active lead can housing 530.

Figure 6 shows a schematical cross-sectional view of the electronic module 500 according to a first embodiment and as shown in Figures 4 and 5.

The electronic module 500 comprises a filtering element, wherein the filtering element is a feed-through filter 567, and a blocking element, wherein the blocking element is a DC blocking element 564 and an ASIC 600, wherein especially the integrated passive device comprises the at least one filtering element, wherein the filtering element is exemplarily a feed-through filter, and/or the at least one blocking element, wherein the blocking element is exemplarily a DC blocking element.

The filtering can be provided by any means which is/are capable to provide a filtering. In particular, a filtering can be provided by any passive means or passive network.

The filtering element may be configured such that interferences, in particular unwanted interferences e.g. caused by mobile phones or the like, can be removed before the may enter e.g. a part of the housing for the electronics of the system for neural applications 100. Thereby, the advantage is achieved that a protection of the interior electronics against electromagnetic interference (EMI) is provided, for example, against mobile phone induced fields while the patient is using its mobile phone. The other way around, (high-frequency) interference generated inside the active lead can 111 is prevented to radiate outside.

The filtering element may be or may comprise e.g. an RF feed-through filter 567. The filter may comprise e.g. a capacitor, a coil, an inductor, a resistor or any other suitable passive component.

The blocking element may be configured such that in the event of a leakage current such leakage current, in particular DC leakage current flow is prevented. Regulations demand that (almost) no DC current flows through the patient carrying an implant such as a deep brain stimulator, even when a (single) failure occurs of, for example, the implant's electronics. This DC leakage design problem is solved by the application of DC blocking capacitors 564. Again, with a high number of feed-through pins 511, 522, it becomes mandatory to integrate those blocking capacitors 564 to achieve a minimum volume and area claim as opposed to the application of discrete components.

The ASIC 600 may comprise a part or e.g. all active electronics with some external passives, for example, power supply decoupling capacitors 800.

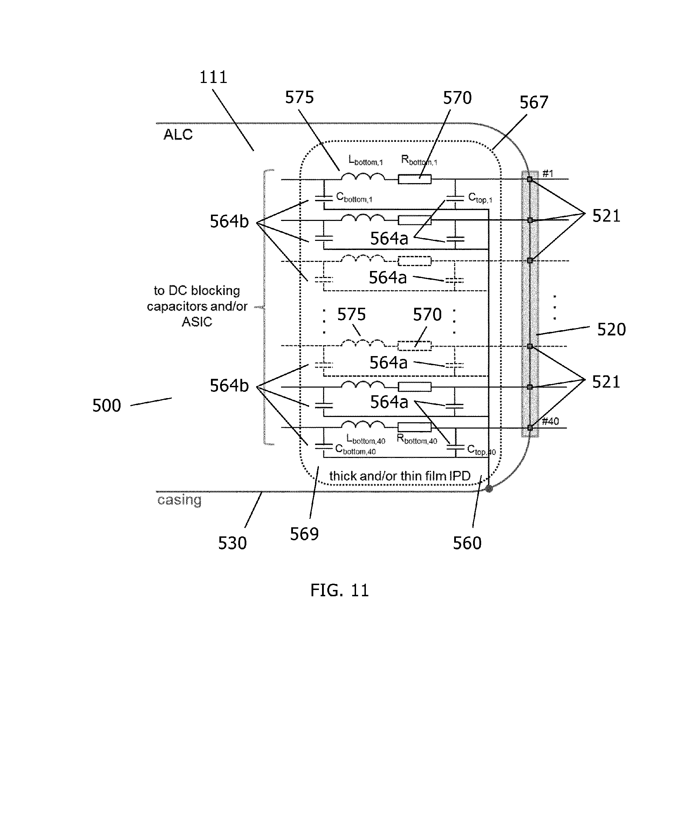

A substrate with integrated passives, here the integrated passive device 560, with e.g. resistors 570, capacitors 564a, 564b and 564c and inductors 575 (see e.g. Figures 11 and 12) may be used as substrate for off-chip (rerouting to) an ASIC 600. By the use of one or more application specific integrated circuits 600 the active electronic components of at least a part of the system for neural application 100 may be miniaturized.

Both for the novel feed-through filter 567 and the 3D silicon DC blocking capacitor technology a substrate is used for their realization, which opens up another unique opportunity to combine all electrical and electronics components into a single stack mounted on top of the feed-through pins 511 of the LCFX connector 510 and the feed-through pins 521 of the HCFX connector 520 directly as shown in Figure 6. This 3-layer stack with the ASIC 600 on the DC blocking element 564 achieves a very high integration density.

Electrical connections are provided via stud bumps 505, which are embedded into epoxy underfill 700.

All active components of the implantable electronic device 111 (e.g., the ASIC 600) and passive (e.g. feed-through filters 567 and DC blocking capacitors 564) components of the active lead can 111 can be combined into a single stack and the stack can be mounted directly on top of the pins 521 of the HCFX connector 520 and the 5-pin 511 LCFX connector 510 (drawing is not true to scale).

Figure 7 shows a further embodiment of the electronic module 1500, which is almost identical with the embodiment shown in Figure 6 but without the DC blocking element 564 and the ASIC 600. All other features are identical and denoted with the same reference numbers.

Figure 8 shows a further embodiment of the electronic module 2500, which is almost identical with the embodiment shown in Figure 6 but without the ASIC 600. All other features are identical and denoted with the same reference numbers.

Figure 8 shows a cross-section of the active lead can 111 interior with all DC blocking capacitors integrated onto a single integrated passive device 568 connected with stud bumps 505 to the (thick film) feed-through filter (array) 567. There is a lot of freedom for signal routing, because thick film interconnect layers can be realized on the (alumina) substrate and/or integrated on the integrated passive device 560 substrate. Thus it is also possible to apply bond wiring instead of stud bumps to connect the feed-through filters with the DC blocking capacitors, because all connections can be routed to bond pads placed on the edges of both the filter substrate 560 bottom and DC blocking capacitor array substrate 568 top.

Figure 13 shows the cross-section of a silicon based integrated passive device 560 that integrates all DC blocking capacitors 564 on a single die. Each capacitor 564 is a 3D trench capacitor realized in the substrate 568 itself (left picture). The capacitors are junction isolated and all diodes 565 share the common substrate 568 (right picture), and therefore, the substrate needs to be biased at an appropriate voltage so that all diodes 565 remain reversed biased during stimulation and recording.

Note that the (back-end) interconnect layers to signal and substrate bias pads are not shown in Figure 13.

The integrated passive device 3D trench capacitors 564 match very well, their leakage performance is very good as well as their reliability and stability. Other passives can also be realized with this type of integrated passive device, although not always on the same substrate, for example, Transient Voltage Suppression (TVS) diodes to handle ESD, metal-insulator-metal (MIM) capacitors, resistors, integrated inductors, etc.

In the future, it might be possible to print feed-through capacitors 567 on the back side of a silicon integrated passive device substrate connected via substrate vias to the trench DC blocking capacitors on the other side. This would reduce the stack to a single, even thinner, integrated integrated passive device containing both the feed-through capacitors 567 as well as the DC blocking capacitors 564.

The fact that the active lead can 111 contains active electronics automatically leads to the need for an array of DC blocking capacitors 564 to prevent any potential DC current flow through the body and the feed-through filter 567 array as barrier for interferers (EMI).

Figure 9 shows a further embodiment of the electronic module 2500, which is almost identical with the embodiment shown in Figure 6 but with discrete surface mounted devices 800 (SMDs 800) mounted onto the DC blocking element 564. All other features are identical and denoted with the same reference numbers.

Figure 9 shows that all circuitry can be combined in a single 3-layer stack with the ASIC 600 on top. This 3-layer stack forms an advanced hybrid with a very high integration density having a minimum volume and area claim.

If not all passive components are integrated in one or more integrated passive devices, the bottom of the silicon substrate 568 can be used as a (routing) substrate 569a for these components, for example realized by the shown discrete surface mounted devices (SMDs) 800. For example discrete DC blocking capacitors 564 that are too large to efficiently integrate in silicon, can be soldered with solders 506 on the bottom side of the silicon integrated passive device substrate 568 together with the ASIC 600.

Note that one could also resort to wire bonding instead of stud bumping to electrically connect the different layers of the active and passive stack of the active lead can 111.

The state-of-the-art feed-through capacitors that are integrated with the feed-through pins themselves and that are today's industry's standard cannot be used for the high pin count of the active lead can 111 because:
1. their pin pitch is too large to match the pin density of an active lead can 111 and it is not possible to take full advantage of the mechanically achievable miniaturization of the feed-through connectors;
2. even more importantly, the application of these state-of-the-art feed-through capacitors makes the active lead can 111 too thick;
3. and they are also not readily available in relatively low capacitance values which lead to unacceptable interface power consumption.

A cross-section of (part of a) feed-through capacitor 567 array that does meet the stringent active lead can volume and area requirements is shown in Figure 10. It can be realized with thin and/or thick film technology and/or with organic capacitors integrated into a flex foil or PCB. If realized in a thick film technology, an (alumina) substrate is used as carrier for the thick film layers printed on both sides of the substrate.

The feed-through capacitors 564a, 564c are (screen) printed on the top side of the substrate 568. The capacitors 564a, 564b and 564c are made of a sandwich of two (or more) conductive layers with a (relatively) high-k dielectric 581 in between. The printed top metal layer, preferably gold, provides the contacts 507 that are wire-bondable or appropriate for stud bumping so that stud bumps 505 and a conductive adhesive epoxy 715 can be used to reliably electrically connect the capacitor array to the feed-through pins 511, 521 of both LCFX and HCFX connector as shown in Figures 6 to 9. At the bottom of the substrate, one or more low-k crossover dielectric sections 582 may be provided.

Figure 10 shows a cross-section of the feed-through filter 567 with a feed-through capacitor array 580, containing capacitors 564a, 564b and 564c, in thick film technology. Each of the feed-through pins 511, 521 connects to a single capacitor in the array via stud bumps 505 on its (gold) top contact 507. Through-hole signal vias 583 connect the feed-through pins 511, 521 to one or more interconnect layers 584 on the bottom side of the substrate 568.

There are two important design aspects of the feed-through capacitor array 580 that must be met to guarantee the filter's interference suppression efficacy:
1. Each metal layer should have a maximum area fill factor so that a maximum overlap between all metal layers is achieved, increasing the capacitance per printed dielectric layer, and forming a (high-frequency) closed Faraday cage with the (titanium) housing 530 of the active lead can 111.
   Thus a minimum spacing between the individual top contacts 507, and therefore a minimum spacing between the through-hole vias 583 and the ground plane 585 increase the filter's efficacy shown in Figure 10, because it increases the capacitance per pin and more importantly, it minimizes the openings in the metal layers. Note that if desired, an additional ground plane 585 can be applied to electrically close the filter's 3D structure further.
2. The mutual ground plane 585 of all array capacitors extends beyond the capacitor array 580 of the LCFX connector 510 and HCFX connector 520 itself and is connected with a ring of ground vias 583a to the opposite side of the substrate to provide ground to the interior of the active lead can 111. Alternatively or additionally, the top and bottom substrate ground planes 585 can be connected via metallization wrapped around the ceramic substrate's 569 side edges.

The ground plane 585a on the substrate top is electrically connected to the housing 530 of the active lead can 111 via a ring of conductive adhesive epoxy 710 as shown in the top view in Figure 5 and individually by the conductive epoxy (ring) 712 for the LCFX and the conductive epoxy (ring) 714 for the HCFX in Figures 6 to 9, so that the feed-through capacitor array 580 and the housing 530 together form a (high-frequency) closed, miniaturized Faraday cage only penetrated by the feed-through pins 511, 521 from which interference signals (EMI) are diverted through feed-through capacitors 564a, 564b and 564c to the (titanium) housing before they can enter the housing itself.

Note that in Figure 10, potentially, the capacitors in the array 580 can be designed with multiple ground-dielectric-signal layers to increase the capacitance/pin and/or enhance the efficacy of the (high-frequency) Faraday cage formed by housing 530 of the active lead can 111 and feed-through capacitor array 567.

The capacitor array 580 on the substrate top can be part of a feed-through filter 567 extended with components printed on the substrate bottom as schematically shown in Figure 11. A series resistor (R_{bottom}) 570 and/or inductor (L_{bottom}) 575 can be added to dampen any (parasitic) filter resonances and/or to increase the (series) impedance of the feed-through filter towards the electronics it is connected to. By this, the (high-frequency) shorting efficacy of the top capacitors (Cₜₒₚ) 564a of the feed-through filter to the housing may be improved. Preferably, another array of capacitors (C_{bottom}) 564b is printed on the substrate bottom to divert any remaining interference current to the housing.

As shown in Figures 11 and 12, the integrated passive device 560 may comprise series resistors 570, series inductors 575 and/or parallel capacitors 564b, wherein the resistors 570, the inductors 575 and/or the capacitors 564b are configured such that a (parasitic) filter resonance may be dampened and/or the filter's suppression, especially the suppression of electromagnetic interference, may be improved and/or increased.

For example, the series resistor 570, the inductor 575 and/or the parallel capacitor 564b can be added to dampen any (parasitic) filter resonances and/or to increase the filter's suppression, especially the suppression of electromagnetic interference. By this, the (high-frequency) shorting efficacy of the filter to the housing 530 may be improved.

The embodiment shown in Figures 11 and 12 relates to the embodiments shown in Figures 4 to 6, but could be also realized in total with the structure of the embodiment of the electronic module 3500 shown in Figure 9 or similarly with the structure of the embodiments of the electronic module 1500, 2500 shown in Figures 7 and 8.

In particular, Figure 11 shows a feed-through filter 567 with the top substrate capacitor array (Cₜₒₚ) extended with series inductor (L_{bottom}) 575, series resistor (R_{bottom}) 570 and another capacitor array (C_{bottom}) 564b printed on the substrate 568 bottom (HCFX connector 520 shown only).

If desired, another capacitor array (C_{bottom}) can be realized on the substrate bottom to suppress any remaining interference. This can be realized with thick or thin film technology on a ceramic substrate with vias, or one could resort to LTCC technology to realize the feed-through filter 567. LTCC stacks layers to form a substrate. The substrate is build out of green tape, where for each filter component (resistor 570, capacitors 564a, 564b, 564c and inductor 575) the most suitable green tape can be selected.

Note that although Figure 11 shows the HCFX connections 520 only, a similar filter (strategy) can be applied at the LCFX connector 510 of both the active lead can 111 and the implantable pulse generator 110.

Figure 12 shows an array of DC blocking capacitors 564 (C_{DC}) in-between ASIC 600 and HCFX filter array 567.

Regulations demand that (almost) no DC current flows through the patient carrying an implant such as a deep brain stimulator, even when a (single) failure occurs of, for example, the implant's electronics. This DC leakage design problem is solved by inserting DC blocking capacitors 564 (C_{DC}) in series with all the implant's feed-through pins and/or feed-through filters as shown schematically for the HCFX connector 520 in Figure 12.

The blocking capacitors 564 are also applied on the LCFX side, although not shown in Figure 12, with a (potential) exception of the interface ground line between active lead can 111 and implantable pulse generator 110 which can directly be connected to the ASIC 600 and housing 530 being the housing of active lead can 111. The housing 530 is a conductive casing 530, preferably a titanium casing 530.

The volume and area claim of the DC blocking capacitors 564 become dominant again if an implant (module) such as the active lead can 111 has a high number of feed-through pins 511, 521. Even worse, the application of discrete capacitors, for example discrete surface mounted SMDs 800 (multi-layer ceramic capacitors (MLCCs); see Figure 9), leads to a prohibitively large active lead can 111 enclosure with complex PCB interconnect.

Figure 13 (left side) shows the cross-section of a typical silicon substrate based integrated passive device 560 with multiple junction isolated passive components (C₁, C₂, ...) in a common substrate 568 having a substrate terminal 566. Each component is junction isolated from the common integrated passive device 560 substrate 568. The back-end interconnects layers and detailed view of each component has been omitted.

As can be further seen in Figure 13 (right side), the integrated passive device 560 with multiple junction isolated passive components (C₁, C₂, ...) may e.g. comprise capacitors 564 and diodes 565 as passive electronic components.

Figure 13 (right side) shows an example of the equivalent electrical circuit of an integrated passive device 560 where each component (C₁, C₂, ...) is an integrated capacitor 564. The integrated passive device 560 has N input terminals (in₁, ..., in_{N}) and N output terminals (out₁, ..., out_{N}) and a single substrate terminal 566 that connects to all component-substrate junctions simultaneously. Resistance as part of interconnect, components and substrate has been left out of the equivalent circuit for simplicity. So, Figure 13 (right side) shows an integrated passive device 560 equivalent electrical circuit for an integrated capacitor array wherein the single substrate terminal 566 is connected to all component-substrate diodes 565 simultaneously.

This invention can be used in all active implantable medical devices that contain active electronics with connections to the body, where the mechanical miniaturization is hampered by the volume and/or area claim of the passive electrical components and networks, in particular, when the active implantable medical device has a high feed-through pin density.

## Claims

1. An electronic module (500; 1500; 2500; 3500) for a system for neural applications (100), especially a neurostimulation and/or neurorecording system (100), further especially a deep brain stimulation (DBS) system (100), wherein the electronic module (500; 1500; 2500; 3500) comprises at least one integrated passive device (560).

2. The electronic module (500; 1500; 2500; 3500) according to claim 1,
**characterized in that**
the electronic module (500; 1500; 2500; 3500) comprises at least one filtering element, wherein the filtering element is exemplarily a feed-through filter (567), and/or at least one blocking element, wherein the blocking element is exemplarily a DC blocking element (568) and/or at least one application specific integrated circuit (ASIC) (600), wherein especially the integrated passive device (560) comprises the at least one filtering element, wherein the filtering element is exemplarily a feed-through filter (567), and/or the at least one blocking element, wherein the blocking element is exemplarily a DC blocking element (568).

3. The electronic module (500) according to claim 1 or 2,
**characterized in that**
the integrated passive device (560) comprises and/or is connected and/or connectable to several sections of interconnects e.g. one or more low-count feed-through pins (511) and/or one or more high-count feed-through pins (521) and/or an ASIC (600) and/or another integrated passive device and/or at least one bias terminal, especially wherein the in- and/or output of the integrated passive device is connected to the filtered out- and/or input via at least one capacitor (564, 564a, 564b, 564c) and/or at least one resistor (570) and/or at least one inductor (575), and/or that the integrated passive device comprises a substrate (568) and at least one diode (565) and/or at least one passive electronic component which is arranged on and/or in the substrate.

4. The electronic module (500) according to one of the preceding claims, especially according to claim 3,
**characterized in that**
the electronic module (500) comprises at least one first connector element (510) and at least one second connector element (520), the first connector element (510) being configured such that the electronic module (500) is directly and/or indirectly connectable or connected to a controller (110) which is at least configured to supply and/or provide and/or measure at least one voltage and/or at least one current and/or at least one voltage waveform and/or at least one current waveform especially via one or more stimulation and/or recording and/or clock and/or power and/or communications outputs and/or inputs (521), the second connector element (520) being configured such that the electronic module (500) is directly and/or indirectly connectable or connected to a lead (300) for neural stimulation and/or recording, and/or the electronic module (500) is directly and/or indirectly connectable or connected to an active lead can element (111) and/or directly and/or indirectly connectable or connected to a housing (530).

5. The electronic module (500) according to one of the preceding claims,
**characterized in that**
the integrated passive device (560) comprises one or more passive electronic components (564, 565, 566), especially at least one capacitor (564) and/or at least one inductor and/or one diode (565) and/or at least one substrate terminal (566), further especially only capacitors and/or inductors and/or diodes and/or at least one substrate terminal (566), especially only one or more passive electronic components (564, 565, 566), especially at least one capacitor (564) and/or at least one inductor and/or at least one diode (565) and/or at least one substrate terminal (566), further especially only capacitors and/or inductors and/or diodes and/or at least one substrate terminal (566).

6. The electronic module (500) according to one of the preceding claims,
**characterized in that**
the integrated passive device (560) comprises one or more feed-through capacitors (564a, 564b, 564c) which are mounted on and/or integrated into a substrate (567), in particular a ceramic substrate (569) and/or one or more integrated DC blocking capacitors (564) which are mounted on and/or integrated into a substrate (568), in particular a silicon substrate (568).

7. The electronic module (500) according to one of claims 3 to 6,
**characterized in that**
the substrate (569) comprises a contact surface portion (585), especially a ground plane (585) on the substrate top, which is directly and/or indirectly electrically and/or capacitively connected to a housing (530) of the electronic module (500), especially via a ring of conductive adhesive, preferably conductive adhesive epoxy (715, 712, 714), so that the feed-through capacitors (564a, 564b, 564c) and the housing (530) together form a (high-frequency) closed, miniaturized Faraday cage only penetrated by feed-through pins (511, 521) that are capacitively and/or electrically coupled to the Faraday cage.

8. The electronic module (500) according to claim 7,
**characterized in that**
the substrate comprises holes (583), wherein through-hole signal vias connect the feed-through pins (511, 521) to one or more interconnect layers on the bottom side of the substrate (569).

9. The electronic module (500) according to one of the preceding claims,
**characterized in that**
the electronic module (500) comprises at least partially a multi-layer structure, wherein especially the filtering element (567) forms a first layer and/or the blocking element (568) forms a second layer and/or the application specific integrated circuit (ASIC) (600) forms a third layer.

10. The electronic module (500) according to one of the preceding claims,
**characterized in that**
the module (500) comprises a dedicated routing substrate and/or an already available substrate for routing (569a) for passive components which are mounted outside an integrated passive device (560), in particular for at least one discrete component, e.g. a surface mounted device (SMD) (800).

11. The electronic module (500) according to one of the preceding claims,
**characterized in that**
the integrated passive device (560) comprises at least one resistor (570), especially a series resistor (570), and/or an inductor (575), especially a series inductor (575), and/or a capacitor (564a, 564b, 564c), especially a parallel capacitor (564a, 564b, 564c), wherein the resistor (570) and/or the inductor (575), and/or a capacitor (564a, 564b, 564c) is/are configured such that a (parasitic) filter resonance may be dampened and/or the filter's suppression, especially the suppression of electromagnetic interference, may be improved and/or increased.

12. A lead (300) for neural stimulation comprising at least one electronic module (500) for a system for neural applications (100) according to any of claims 1 to 11.

13. A controller (110), especially an implantable pulse generator (110), comprising at least one electronic module (500) for a system for neural applications (100) according to any of claims 1 to 11.

14. An active lead can element (111) comprising at least one electronic module for a system for neural applications (100) according to any of claims 1 to 11.

15. A system for neural applications (100), especially a neurostimulation and/or neurorecording system (100), especially a deep brain stimulation (DBS) system (100), comprising at least one electronic module (500) for a system for neural applications (100) according to any of claims 1 to 11 and/or comprising at least one lead (300) according to claim 12 and/or comprising at least one controller (110) according to claim 13 and/or comprising at least one active lead can element (111) according to claim 14.
